(19) Europäisches Patentamt / European Patent Office / Office européen des brevets

(11) **EP 3 907 486 A1**

(12) **EUROPÄISCHE PATENTANMELDUNG**

(43) Veröffentlichungstag:
**10.11.2021 Patentblatt 2021/45**

(51) Int Cl.:
**G01N 7/14** (2006.01) **G01N 33/14** (2006.01)
**G01N 21/552** (2014.01)

(21) Anmeldenummer: **21171763.2**

(22) Anmeldetag: **03.05.2021**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **06.05.2020 AT 503842020**

(71) Anmelder: **Anton Paar GmbH**
**8054 Graz-Straßgang (AT)**

(72) Erfinder: **BLODER, Josef**
**8200 Gleisdorf (AT)**

(74) Vertreter: **Wildhack & Jellinek**
**Patentanwälte OG**
**Landstraßer Hauptstraße 50**
**1030 Wien (AT)**

(54) **VERFAHREN UND VORRICHTUNG ZUR BESTIMMUNG DES GEHALTS EINES FREMDGASES IN EINER PROZESSFLÜSSIGKEIT**

(57) Verfahren und Vorrichtung zur Bestimmung des Gehalts eines Fremdgases in einer Prozessflüssigkeit, in dem ein Messgas, insbesondere $CO_2$, gelöst ist, wobei die Konzentration des Messgases ermittelt wird, - wobei die Konzentration des durch das Messgas und das Fremdgas gebildeten Gasgemischs, insbesondere über eine manometrische Messmethode, ermittelt wird, - wobei die Messwerte einer Auswerteeinheit zugeführt werden, - wobei anhand der ermittelten Konzentration des Messgases und der ermittelten Konzentration des Gasgemisches die Konzentration des Fremdgases bestimmt wird.

Fig. 1

EP 3 907 486 A1

**Beschreibung**

Die vorliegende Erfindung betrifft ein Verfahren zur Bestimmung des Gehalts eines Fremdgases in einer Prozessflüssigkeit gemäß dem Patentanspruch 1 sowie eine Vorrichtung zur Durchführung des erfindungsgemäßen Verfahrens gemäß dem Oberbegriff des Patentanspruchs 13.

Aus dem Stand der Technik ist die Bestimmung von Gasen in Fluiden aus der Messung von Druck und Temperatur des spezifischen Gases bekannt. Für die Bestimmung der Konzentration von Gasen, die in Flüssigkeiten gelöst sind, wie insbesondere Kohlendioxid in Getränken, ist eine größere Anzahl von oft recht unterschiedlichen Verfahren und Vorrichtungen bekannt, die kommerziell verfügbar sind und verwendet werden.

So sind beispielsweise manometrische Verfahren zur Druck- und Temperaturmessung bekannt, bei denen nach Einstellung bzw. Herstellung des Gleichgewichts zwischen zu untersuchenden Fluid und darüber befindlichen Kopfraum, die Temperatur und der Druck gemessen werden. Die Konzentration des Messgases beispielsweise Kohlendioxid $CO_2$ wird dann mithilfe einer modifizierten Henry-Dalton-Gleichung (Gl.1) (Siehe "Die manometrische Bestimmung des CO2-Gehalts in Getränken", Brauwelt 50, 1991, p.2402 ff.) bestimmt:

$$c_{CO2} = \xi_{CO2} * (p_{ges} - p_{kor}) + c_{kor} \qquad (Gl.1)$$

mit

$c_{CO2}$ in g/L (Konzentration)
$\xi_{CO2}$ in g/L/bar (Löslichkeit)

In der modifizierten Dalton-Gleichung für Gase in Gebinden wird hier eine zusätzliche Schütteldruckkorrektur $p_{kor}$ und Konzentrationskorrektur $c_{kor}$ angewandt.

Die Löslichkeit $\xi$ ist eine stoffspezifische Größe, die mit steigender Temperatur sinkt.

Die Menge des absorbierten Gases in einer Prozessflüssigkeit ist dabei abhängig von der spezifischen Löslichkeit, dem Druck und der Temperatur.

Bei der Anwendung der Henry-Dalton-Gleichung liegt das Messgas, insbesondere Kohlendioxid, im Gleichgewichtszustand zwischen Getränk und darüber liegender Gasphase vor. Bekannt ist dabei, dass bei dieser Methode Fremdgase das Messergebnis verfälschen. Im Gleichgewichtszustand eines Gasgemisches kann jede gelöste Gaskomponente i über den jeweiligen Anteil des Partialdruckes wie in Gl. 2 angegeben bestimmt werden:

$$c_i = \xi_i(T) * p_i \qquad (Gl. 2)$$

Somit addieren sich die Partialdrücke der Fremdgase mit dem tatsächlichen Partialdruck des Kohlendioxid zum Gesamtdruck. Als Fremdgase werden bei Getränken insbesondere alle Gase außer Kohlendioxid, beispielsweise Stickstoff, Sauerstoff, Lachgas verstanden, die entweder auf Grund des Produktionsprozesses entstehen, nicht eliminiert oder bewusst zugefügt werden.

Ein manometrisches Verfahren zur Bestimmung des Drucks und der Temperatur in einem Getränk wird beispielsweise mittels der Expansionsmethode durchgeführt. Bei der Expansionsmethode wird eine repräsentative Probe der Messflüssigkeit in eine Messkammer eingeführt bzw. in der Prozesslinie direkt in eine Messkammer entnommen. Bei Messungen an Getränkegebinden dient häufig das ganze Gebinde als Messkammer. Nach dem Verschließen der Messkammer wird die zu untersuchende Flüssigkeitsprobe durch Vergrößerung des Volumens der Messkammer, z.B. mittels einer Art von an die Messkammer fluiddicht angeschlossener Kolbenspritze oder Membran und bei der Messung in Gebinden durch kurzzeitige Druckentlastung, entspannt. Der sich nach dem Entspannen einstellende Druck und die Probentemperatur werden dann gemessen. Daraus wird nach dem Henry Gesetz der Kohlendioxidgehalt berechnet.

Wird die Probe der Flüssigkeit in der abgeschlossenen Messkammer entspannt, so bilden sich aus der ursprünglich reinen flüssigen Phase, in der alle Gase, also das Messgas und auch die Fremdgase gelöst sind, eine flüssige und eine Gas-Phase. Wenn die Gase stark unterschiedliche Löslichkeiten in der Probeflüssigkeit aufweisen, dann unterscheidet sich das Verhältnis der Partialdrücke der einzelnen Gase in der Gas-Phase wesentlich vom Verhältnis der Sättigungsdrücke der gelösten Gase in der ursprünglich - also vor der Expansion - vorhandenen Probeflüssigkeit. Generell gilt, dass der Partialdruck eines in einer Flüssigkeit gelösten Gases bei einer Volumen-Vergrößerung umso stärker abnimmt, je kleiner seine Löslichkeit in der Flüssigkeit ist.

Im Falle von Getränken, z.B: Bier oder Softdrinks in einer Prozesslinie reicht zur Messung des CO2-Gehalts die einfache Volumenvergrößerung zumeist aus, um die Menge an gelöstem CO2 zu bestimmen, der Anteil von Fremdgasen, wie beispielsweise Luft also Sauerstoff und Stickstoff, ist hier meist vernachlässigbar. Die Volumenvergrößerung liegt dabei der Messung nach der Expansionsmethode bevorzugt im Rahmen von 3 % bis 10 % des Ausgangsvolumens. Bei dieser Methode wird also primär der Sättigungsdruck des Kohlendioxids in der zu analysierenden Flüssigkeit ermittelt. Sonstige in der Probeflüssigkeit gelöste Gase, wie insbesondere Sauerstoff und auch Stickstoff, beeinflussen jedoch den ermittelten Kohlendioxidgehalt. Die derzeit kommerziell verfügbaren Vorrichtungen unterscheiden sich voneinander u. a. durch die Art der Entnahme der Probe, die Gestaltung der Messkammer und durch verschiedene Maßnahmen zur beschleunigten Einstellung des Gleichgewichts, wie beispielsweise Rührwerke etc.

Vernachlässigt man den Fremdgas- bzw. Stickstoffgehalt nicht oder setzt dem Getränk sogar Stickstoff zu, was das sogenannte Mundgefühl bei bestimmten Biersorten verbessert, limitiert das die Anwendbarkeit der einfachen Expansionsmethode. Insbesondere in irischen Brauprodukten und/oder Softdrinks wie Energydrinks und /oder Limonaden wird zusätzlich Stickstoff oder Lachgas hinzugefügt. Das resultierende Bier hat weniger Kohlensäure, das Mundgefühl ist weicher und voller, die geschmacklichen Noten werden abgerundet, aber auch etwas abgeschwächt. Diese zusätzliche Abrundung wird vor allem bei malzig-röstigen Bieren durchgeführt. Damit wird die Stickstoffbeigabe mit dunkleren Sorten auch für Bierbrauer und Abfüllanlagen zunehmend interessant. Der Stickstoffgehalt des Prozessbiers in der Produktionslinie steigt, womit auch der Einfluss des Stickstoffs auf die CO2-Messung nicht mehr vernachlässigbar ist. Bei Soft Drinks bzw. Limonaden gibt es Getränke bei denen statt reinem $CO_2$ ein Gemisch aus $CO_2$ und $N_2O$, Distickstoffmonoxid auch als Lachgas bezeichnet, zugesetzt wird.

Aus der AT409673 (B) der Anmelderin selbst wird im Stand der Technik ein Verfahren für die Messung von zumindest zwei unterschiedlichen in dem Fluid enthaltenen Gasen und ein Verfahren zur Bestimmung der Gehaltsmengen in der Flüssigkeit, vorzugsweise in einem Getränk, gelösten Gasen offenbart. In der AT409673 (B) werden die Gase aus einer Mehrfachexpansion des Messvolumens und zugehöriger Druckmessung ermittelt. Bei dem Verfahren wird nach vollständigem Befüllen der Messkammer mit der auf ihren Gasgehalt zu prüfenden Flüssigkeit ("Probeflüssigkeit") deren Volumen - von einem Standardvolumen ausgehend - um einen vorgegebenen Faktor vergrößert und der sich danach in der Messkammer einstellende Gleichgewichtsdruck ermittelt und - basierend auf dem so erhaltenen Druckmesswert - der Gasgehalt des Messgases in der zu untersuchenden Flüssigkeit berechnet. Anschließend wird das Volumen in zumindest zwei bzw. mehrere Expansionsschritte weiter vergrößert. Nach jedem der Volumenvergrößerungsschritte wird der sich in der Messkammer jeweils einstellende Gleichgewichtsdruck ermittelt und daraus über ein Gleichungssystem die Konzentration/Gehaltsmengen der einzelnen Komponenten berechnet.

Bei mehreren gelösten Gasen sind also entsprechend viele VolumenVergrößerungsschritte und entsprechend umfangreichere Berechnungsverfahren notwendig. Ist mehr als ein Gas in der Prozessflüssigkeit gelöst und sind auch die Löslichkeiten einzelner Gase zu ermitteln, so ergeben sich nichtlineare Gleichungssysteme höherer Ordnung, deren Lösung erfolgt typischerweise iterativ, ausgehend von möglichst realitätsnah genauen Schätzwerten für die unbekannten Gas-Löslichkeiten. Vergleicht man die Löslichkeiten von Kohlendioxid und Stickstoff in Getränken, so ist diese stark unterschiedlich, und der sich ergebende Partialdruck unterscheidet sich wesentlich, was die Ermittlung des Stickstoffgehaltes mit Hilfe einer komplexen Mathematik erst möglich macht. Die Ermittlung von Distickstoffmonoxid in einem Getränk mit Kohlendioxid und Distickstoffmonoxid ist mit diesem Verfahren nicht möglich, da die Löslichkeit von Distickstoffmonoxid und Kohlendioxid nahezu gleich sind.

Weiters sind aus dem Stand der Technik Verfahren zur Konzentrationsbestimmung eines Gases bekannt, bei der das Gas selbst ohne den Einfluss von Fremdgasen bestimmt wird. Dies ist beispielsweise durch eine optische ATR-Messung möglich. Das Verfahren beruht auf der Auswertung von Absorptions- bzw. Transmissionsspektren, in denen die Anregung von charakteristischen Molekülschwingungen, dies sind Rotation und/oder Vibration, in der Flüssigkeit zu einer Energieabsorption und damit einer Intensitätsänderung im anregenden Spektrum führt. Mit diesem Verfahren können Inhaltsstoffe geringer und geringster Konzentration ermittelt werden, wobei aus der Absorption von Infrarotstrahlung die jeweilige Konzentration des Inhaltsstoffs in festen, flüssigen oder gasförmigen Medien ermittelt wird. Je nach Messaufgabe werden unterschiedliche Wellenlängenbereiche des Spektrums für die Strukturaufklärung eingesetzt, der Messbereich reicht von UV/VIS- bis hin in den Infrarotbereich. Aus der Absorption von Strahlung bestimmter Energie kann auf die angeregten Molekül- bzw. Gitterschwingungen und damit auf die Bestandteile des untersuchten Materials rückgeschlossen werden. Für die Messstrahlung hinreichend durchlässige Substanzen können in Transmission vermessen werden, für opake Festkörper und für stark gefärbte Lösungen ist die Untersuchung der Reflexion wie beispielsweise mit der Methode der abgeschwächten Totalreflexion (ATR) bekannt. In der Prozessanalytik ist die Anwendbarkeit von Transmissionsmessungen häufig durch die starke Absorption durch Wassermoleküle im infraroten Bereich begrenzt, sodass Reflexionsmessungen wie die Methode der ATR vorteilhaft zur Anwendung gelangen. Die spektroskopische Bestimmung zeigt auch den Vorteil, dass die Messergebnisse unabhängig vom Druck der untersuchten Flüssigkeit sowie deren Komponenten sind. Die optische Messung zeigt also keine Querempfindlichkeiten gegenüber anderen als den zu Messenden Stoffen.

Die AT512291 (B1) zeigt eine Ausführungsform zur optischen Messung des Gasgehalts von Fluiden, wobei diese Patent auch eine genaue Berücksichtigung von ggf. die Konzentration verfälschenden absorbierenden Extraktanteilen für einen ATR-Sensor korrigiert.

Während optische Messgeräte und einfache Expansionsgeräte problemlos in einer Prozesslinie bzw. in einem Prozess mit einer Prozessflüssigkeit integriert werden können, sind die entsprechenden Geräte zur Mehrfachexpansion und damit der Bestimmung eines Fremdgases in einer Prozessflüssigkeit komplex im Aufbau und können nur im sogenannten Bypass betrieben werden. Weiters ist auch die Reinigung dieser bei den Messgeräten vorhandenen Mehrfachexpansionskammern sehr aufwändig und kann insbesondere im Lebensmittelbereich zu hygienischen Problemen führen.

Insbesondere die Ermittlung der Stickstoffkonzentration als Fremdgas in kohlendioxidhaltigen Getränken ist schwer, da für diese Messung keine geeigneten Messgeräte existieren.

Insbesondere die Ermittlung der Distickstoffmonoxid-Konzentration als Fremdgas in Kohlendioxidhaltigen Getränken ist in der Doppelhubmethode nicht möglich, da die Gase aufgrund der beinahe identen Löslichkeit mit dieser Methode nicht unterscheidbar sind..

Aufgabe der Erfindung ist es daher ein Verfahren bereit zu stellen, mit dem ein Fremdgas in einer Prozessflüssigkeit, in dem ein Messgas, insbesondere $CO_2$, gelöst ist, zu bestimmen. Diese Aufgabe wird durch das erfindungsgemäße Verfahren gemäß Patentanspruch 1 gelöst.

Erfindungsgemäß ist dabei vorgesehen, dass wobei die Konzentration des Messgases ermittelt wird, wobei die Konzentration des durch das Messgas und das Fremdgas gebildeten Gasgemischs, insbesondere über eine manometrische Messmethode, ermittelt wird, wobei die Messwerte einer Auswerteeinheit zugeführt werden, wobei anhand der ermittelten Konzentration des Messgases und der ermittelten Konzentration des Gasgemisches die Konzentration des Fremdgases bestimmt wird.

Erfindungsgemäß wird also eine genaue Messung des Messgases durchgeführt und anschließend eine Messung des Gesamtdrucks und der Temperatur des durch das Fremdgas und Messgas gebildete Gasgemisch durchgeführt. Da der Gehalt des Fremdgases insbesondere von Stickstoff schwer und von Distickstoffmonoxid nicht zu bestimmen ist, wird mit der erfindungsgemäßen Methode der Gehalt des Prozessgases zb der $CO_2$-Gehalt auf zwei unterschiedliche Methoden bestimmt, einmal mit einer exakten Methode, in der die Konzentration des Messgases exakt bestimmt wird und mit einer zweiten Methode, in der die $CO_2$-Konzentration durch das Fremdgas bewusst fehlerbehaftet bestimmt wird. Diese beiden Methoden werden dann zueinander verglichen und derart eine einfache Bestimmung des Fremdgases über den Vergleich der beiden Messwerte erreicht.

Derart wird eine einfache Messmethode bereitgestellt, die es auch erlaubt, innerhalb einer Prozesslinie einfach integriert zu werden und dabei kostengünstig ist und einen einfachen Aufbau aufweist.

Besonders vorteilhafte Ausführungsformen des erfindungsgemäßen Verfahrens werden durch die Merkmale der abhängigen Ansprüche näher definiert:
Eine Vorteilhafte Messung Bestimmung der Konzentration des Fremdgases wird erreicht indem, der Gesamtdruck und die Temperatur des durch das Messgas und das Fremdgas gebildeten Gasgemischs, insbesondere über eine manometrische Messmethode, ermittelt wird, wobei anhand der ermittelten Konzentration des Messgases und des ermittelten Gesamtdrucks und der ermittelten Temperatur der Partialdruck des Messgases bestimmt wird, wobei der Partialdruck des Fremdgases anhand des ermittelten Partialdrucks des Messgases und anhand des gemessenen Gesamtdrucks und der gemessenen Temperatur ermittelt wird, und wobei über den Partialdruck des Fremdgases die Konzentration und/oder der Gehalt des Fremdgases bestimmt wird.

Vorteilhaft kann vorgesehen sein, dass nach der Ermittlung der Konzentration des Messgases der Prozessflüssigkeit Fremdgas hinzugefügt wird.

So kann beispielsweise bei der Produktion von Getränken dem Getränk- bzw. der Prozessflüssigkeit das Fremdgas wie zB Stickstoff oder Lachgas erst im Laufe des Herstellungsprozesses zugeführt werden, wobei der Gehalt bzw. die dann vorliegende Konzentration des Fremdgases über die erfindungsgemäße Methode einfach bestimmt werden kann.

Vorteilhaft kann vorgesehen sein, dass die Konzentration des Messgases mittels eines manometrischen Sensors über eine manometrische Messmethode, insbesondere über eine Expansionsmethode, bevorzugt mit einer Volumsvergrößerung von 1% bis 20%, besonders bevorzugt mit einer Volumsvergrößerung von 3%- 10%, ermittelt wird. Je kleiner die Volumsvergrößerung ist umso schneller ist eine Entgasung durchführbar und damit das Gleichgewicht zwischen Flüssigkeit und Gas erreicht. Die Wahl der Expansionsvolumina erfolgt dabei: je keiner die Expansionsvolumina desto stärker gehen Fehler der Volumsvergrößerung in die Messung ein. Bevorzugt werden daher kleine Volumsvergrößerungen bei dem erfindungsgemäßen Verfahren eingesetzt.

So kann beispielsweise das Messgas bzw. das Gasgemisch aus Messgas und Fremdgas vor Zugabe des Fremdgases über eine manometrische Methode bestimmt werden, dann das Fremdgas der Prozessflüssigkeit hinzugefügt und anschließend noch einmal eine manometrische Messmethode durchgeführt werden und so der Anteil des Fremdgases durch Berechnung der Unterschiede der beiden Messwerte bestimmt werden.

Um eine exakte, also fremdgasunabhängige, Bestimmung des Konzentrationsgehaltes des Messgases, bestimmen zu können, kann vorgesehen sein, dass die Konzerntration des Messgases mittels eines ersten Sensors ermittelt wird, der als optischen Sensor, insbesondere als ATR-Sensor, ausgebildet ist, wobei mittels des ersten Sensors insbesondere eine optische Absorptionsmessung des Messgases durchgeführt wird

Die ATR-Methode ist, wie einleitend beschrieben, bereits gut bekannt und ermöglicht es, die Konzentration beispielsweise von $CO_2$ in den Prozessflüssigkeiten wie Getränken exakt, also ohne Fehler, durch das Fremdgas zu bestimmen und findet daher vorteilhaft Anwendung in dem erfindungsgemäßen Verfahren.

Vorteilhaft kann vorgesehen sein, dass der Gesamtdruck und die Temperatur des durch das Messgas und das Fremdgases gebildete Gasgemischs mittels eines zweiten Sensors ermittelt wird, der als manometrischer Sensor ausgebildet ist und der Gesamtdruck über eine manometrische Messmethode, insbesondere über eine Messung nach der Druck-Temperatur-Methode oder Volumenexpansionsmethode, ermittelt wird.

Eine manometrische Messung des Gesamtdrucks und der Temperatur der durch das Messgas und das Fremdgas gebildete Gasgemisch kann, wie aus dem Stand der Technik bekannt, einfach über eine manometrische bzw. sogenannte Expansionsmethode erfolgen. Insbesondere in Kombination mit einem optischen Sensor beispielsweise einem ATR-Sensor, der den exakten Gehalt des Messgases bestimmt, kann derart die Konzentration des Fremdgases besonders effektiv und mit einem einfachen Aufbau bestimmt werden.

Der Partialdruck des Fremdgases kann einfach ermittelt werden, indem der Partialdruck des Fremdgases ($P_F$) mithilfe der Gleichung $P_F = (p'_{gesamt} - p_{MG}/ (1 + v / L_{MG})) * (1 + v / L_F)$ ermittelt wird.

Insbesondere bei der Vermessung von Getränken bzw. Prozessflüssigkeiten in der Lebensmitteltechnologie kann vorgesehen sein, dass das Messgas Kohlendioxid und das Fremdgas insbesondere Stickstoff oder Distickstoffmonoxid ist.. Damit kann die Fremdgasbestimmung mittels Standardsensoren für ein häufig zu vermessendes Messgas erfolgen.

Um abhängig von der Prozessflüssigkeit den Anteil des Fremdgases und des Messgases noch genauer bestimmen zu können, kann vorgesehen sein, dass bei der Bestimmung der Konzentration des Messgases und/oder des Fremdgases die Löslichkeit und/oder die Kompressibilität des Messgases und/oder des Fremdgases in der Prozessflüssigkeit berücksichtigt wird.

Die Messung bzw. Auswertung der Konzentration des Messgases bzw. des Fremdgases kann weiter verbessert werden, indem dass die Differenz der Messwerte der beiden Sensoren mit Proben bekannter Konzentration von Messgas und Fremdgas kalibriert werden und diese Kalibrationskurven oder Tabellen in der Auswerteeinheit hinterlegt werden.

Die Konzentration des Fremdgases kann besonders einfach bei bekannten und wiederkehrenden Probeflüssigkeiten bestimmt werden, indem der Zusammenhang zwischen der Konzentration des Messgases ohne Fremdgaseinfluss und der Konzentration des Gasgemisches bestehend aus Messgas und Fremdgas vor Beginn der Messung anhand von Messungen an bekannten Proben bestimmt wird, dass die Werte für die Fremdgaskonzentration als, insbesondere temperaturabhängige, Konzentrationsdifferenz des Messgases als Berechnungskurven und/oder Tabellenwerte in der Auswerteeinheit der Messgeräte zur Verfügung gestellt werden, und dass die Fremdgaskonzentration anhand dieser Werte bei der Messung ermittelt wird.

Eine bevorzugte Anwendung des erfindungsgemäßen Verfahrens in der Lebensmitteltechnologie kann erreicht werden, indem die Bestimmung der Konzentration des Messgases und die Messung des Gesamtdruckes und Temperatur des Gasgemisches in einem Leitungssystem der Prozessflüssigkeit oder einem Behälter, insbesondere in geringem Abstand zu einander, bevorzugt einander gegenüberliegend, erfolgt. Damit kann sichergestellt werden, dass die Messbedingung der beiden Sensoren ident sind und insbesondere keine Konzentrationsunterschiede vorherrschen.

Das erfindungsgemäße Verfahren findet besonders vorteilhafte Anwendung bei der Produktion bzw. Kontrolle von Getränken, wobei die Bestimmung von Stickstoff bzw. Lachgas im Getränk, wie zB Bier, mit dem erfindungsgemäßen Verfahren besonders gut ermittelt werden kann.

Insbesondere bei Getränken ist die Prozessflüssigkeit immer wieder mit Extrakten vermischt bzw. enthält Extrakte oder Schwebeteilchen, die die Messwerte verfälschen können. Erfindungsgemäß kann daher vorgesehen sein, dass bei der Bestimmung des Gehalts des Messgases der Gehalt von in der Prozessflüssigkeit enthaltener Extrakte berücksichtigt wird..

Das erfindungsgemäße Verfahren wird weiter verbessert, indem bei der Bestimmung der Konzentration des Messgases und des Fremdgases der Dampfdruck der Prozessflüssigkeit berücksichtig wird. Durch die Berücksichtigung des Dampfdrucks der Prozessflüssigkeit wird die Genauigkeit der Bestimmung der Dampfdrücke von Fremd- und Messgas verbessert.

Eine alternative Anwendung des Verfahrens kann bereitgestellt werden, indem die Prozessflüssigkeit, das Messgas und das Fremdgas in einem Behälter in zweiphasigem Zustand vorliegen, wobei die Prozessflüssigkeit mit gelösten Anteilen des Messgases und des Fremdgases die erste Phase bilden und ein ungelöster Anteil des Messgase und des Fremdgases als Gasgemisch die zweite Phase ausbilden, wobei der erste Sensor und der zweite im Bereich des Behälters angeordnet sind indem das Messgas und das Fremdgas in der Prozessflüssigkeit gelöst vorliegen.

Ein weiterer Aspekt der Erfindung sieht vor, eine Vorrichtung bereitzustellen, mit der das erfindungsgemäße Verfahren durchgeführt werden kann. Diese Aufgabe wird durch die kennzeichnenden Merkmale des Anspruchs 13 gelöst. Erfindungsgemäß ist dabei vorgesehen, dass die Vorrichtung einen ersten Sensor aufweist, mit dem der Gehalt des Messgases ermittelbar ist, und dass die Vorrichtung einen, insbesondere zweiten, Sensor aufweist, mit dem der Gesamtdruck und die Temperatur des Messgases und des Fremdgases in der Prozessflüssigkeit, insbesondere über eine manometrische Messmethode, ermittelbar ist, und dass die Vorrichtung eine Auswerteeinheit aufweist, die zur Durchführung des erfindungsgemäßen Verfahrens ausgebildet ist.

Eine besonders exakte Bestimmung des Messgases kann erreicht werden, indem der Sensor als optischer Sensor, insbesondere als ATR-Sensor, ausgebildet ist.

Vorteilhaft kann vorgesehen sein, dass der Sensor, insbesondere der zweite Sensor, als manometrischer Sensor ausgebildet ist, und insbesondere derart ausgebildet ist, dass die Messung nach der Druck-Temperatur-Methode oder Volumenexpansionsmethode mittels des Sensors durchführbar sind.

Das erfindungsgemäße Verfahren kann besonders einfach durchgeführt werden, indem dass der erste Sensor und der zweite Sensor in dem Leitungssystem in geringem Abstand zueinander, insbesondere gegenüber voneinander, angeordnet sind, und insbesondere über Flansche in dem Leitungssystem anbringbar sind. oder der erste Sensor und der zweite Sensor im Behälter gegenüber einander angeordnet sind.

Weitere Vorteile und Ausgestaltungen der Erfindung ergeben sich aus der Beschreibung und den beiliegenden Zeichnungen.

Die Erfindung ist im Folgenden anhand von besonders vorteilhaften, aber nicht einschränkend zu verstehenden Ausführungsbeispielen in den Zeichnungen schematisch dargestellt und wird unter Bezugnahme auf die Zeichnungen beispielhaft beschrieben:

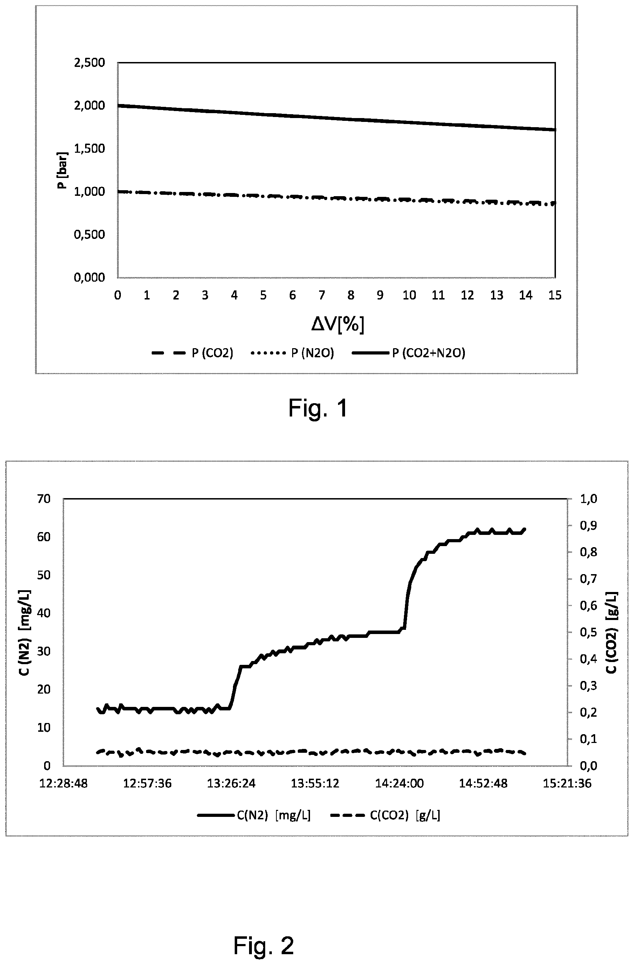

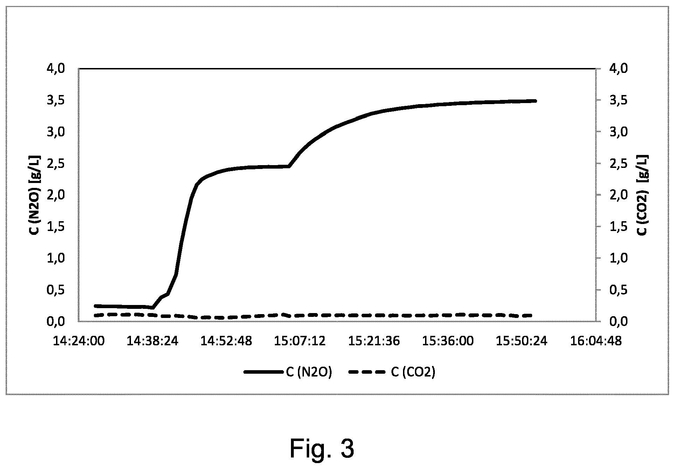

Fig. 1 und 2 zeigen ein Diagramm der Anteile der Partialdrücke von $CO_2$ und den Fremdgasen Stickstoff und Lachgas in Abhängigkeit von der Volumsvergrößung $\Delta V$ bei einer Messung mittels der Expansionsmethode, und Fig. 3 zeigt die Messergebnisse eines experimentellen Versuches zur Bestimmung des Stickstoffgehaltes sowie des Lachgasgehalts in einer Prozessflüssigkeit mithilfe des erfindungsgemäßen Verfahrens.

Im Folgenden werden die erfindungsgemäße Vorrichtung und das erfindungsgemäße Verfahren näher beschrieben. So kann in einem ersten Ausführungsbeispiel die Vorrichtung ein Leitungssystem aufweisen, in dem die Prozessflüssigkeit fließt. In der Prozessflüssigkeit zb Bier ist ein Messgas zB Kohlendioxid, hier in geringer Konzentration gelöst. In dem Leitungssystem ist ein erster Sensor integriert, mit dem der Gehalt des Messgases möglichst genau beispielsweise durch eine optische Messung ermittelt wird. Weiters weist die Vorrichtung einen zweiten Sensor zur Kohlendioxidmessung auf, der beispielsweise als manometrischer Sensor ausgebildet ist, mit dem dann der Gesamtdruck und die Temperatur des Messgases und des Fremdgases, also des aus diesen beiden Gasen gebildeten Gasgemisches, in der Prozessflüssigkeit ermittelt wird. Die Messdaten des ersten Sensors und des zweiten Sensors werden dann einer Auswerteeinheit zugeführt, die aus diesen Messdaten bzw. aus den durch die Sensoren gewonnenen Messwerten den Gehalt des Fremdgases bestimmt.

Erfindungsgemäß wird dabei beispielsweise mittels des ersten Sensors die $CO_2$-Konzentration optisch nach der ATR-Methode ohne Fremdgaseinfluss bestimmt und mit dem zweiten Sensor dann beispielsweise über die Druck- und Temperaturmessung der sogenannten scheinbaren $CO_2$-Gehalt ermittelt und dieser dann mit dem exakt gemessenen $CO_2$-Gehalt verglichen. Aus der doppelten Messung des $CO_2$-Gehaltes wird dann über den Partialdruck bzw. die Konzentrationen der gemessenen $CO_2$-Werte nach dem erfindungsgemäßen Verfahren beispielsweise mittels der Henry-Dalton Gleichung der Partialdruck und damit die Konzentration des Fremdgases ermittelt.

Das erfindungsgemäße Verfahren nutzt einen durch das Fremdgas unbeeinflussten Messwert für die Bestimmung der exakten CO2-Konzentration, der mit einem optischen Sensor zb ATR-Sensor erfasst wird und vergleicht diesen mit dem "fehlerbehafteten" CO2- Konzentrationswert aus der manometrischen Methode, um auf den Partialdruck und die Konzentration des Fremdgases zu schließen.

In der nachfolgenden Ausführungsform des Erfindungsgemäßen Verfahren misst der erste Sensor ein Carbo 520, von Anton Paar (https://www.anton-paar.com/at-de/produkte/details/carbo-520-optical/) die $CO_2$-Konzentration optisch nach der ATR Methode ohne Fremdgaseinfluss. Ein zweiter Sensor zur. Druck- und Temperaturmessung, Carbo 510 von Anton Paar (https://www.anton-paar.com/corp-de/produkte/details/carbo-510-smart-sensor/) ermittelt den scheinbaren oder fehlerbehaltenen $CO_2$-Gehalt aus dem gemessenen Gesamtdruck. Ist kein zweites Gas vorhanden entspricht der scheinbare $CO_2$-Gehalt dem tatsächlichen $CO_2$-Gehalt und es kann derart beispielsweise überprüft werden, ob überhaupt ein Fremdgas in der Prozessflüssigkeit enthalten ist.

Unterscheiden sich die durch den ersten und zweiten Sensor ermittelten $CO_2$-Gehalte kann dann aus der Konzentration und/oder dem bestimmten Partialdruck des $CO_2$ der Partialdruck und die Konzentration des Fremdgases z.B. Stickstoff oder Distickstoffmonoxid anhand der Nachfolgenden Berechnung ermittelt werden.

Bei Anwesenheit eines Fremdgases ergibt sich der mit der manometrischen Methode gemessene Gesamtdruck aus den Summen der Partialdrücke.

Dalton-Gesetz: pgesamt = $\sum p_i$

Für das CO2 und Fremdgas gilt somit: pgesamt = $p_{CO2} + p_{FG}$

Die Konzentration kann dann mit dem Henry Dalton Gesetz bestimmt werden: ci = $\xi i(T)*pi$ ci in g/L (Konzentration eines Gases)

$\xi$ i in g/L/bar (Löslichkeit eines Gases)

Die $CO_2$ und $N_2O$ Konzentration kann auch in der Einheit "Volumen" (Vol) statt "g/L" angegeben werden. 1 Vol

= 1 Liter $CO_2$ oder $N_2O$ unter Standarddruck (1 bar) pro Liter Getränk. (Die Einheiten können über die Gasgleichung umgerechnet werden).

Die CO2 Konzentration errechnet sich dann nach: $c_{CO2V} = p_{CO2} * L_{CO2}$

$c_{CO2V}$ ... CO2- Konzentration in Volumeneinheit

$$L_{COS} \ldots \text{Löslichkeit in Volumen/bar} = \xi /(M/(R*T0*Z0)) = \xi /1.951$$

T0 = 273.2 K, Z0 = 0.993, $M_{CO2}$ = 44.01 g/mol,

R ... Gaskonstante = 8.3145*10-2 [l*bar/(K*mol)]

Aus dem mit dem Fremdgas-unabhängigen optischen Sensor bestimmten CO2-Konzentrationswert kann auf den wahren Partialdruck des CO2 $p_{CO2}$ geschlossen bzw. zurückgerechnet werden.

$$p_{CO2} = c_{CO2OPT} / \xi_{CO2}$$

Aus dem manometrischen Gesamtdruckmesswert für $CO_2$ plus Fremdgas und des tatsächlichen Partialdrucks des $CO_2$, kann sodann auf den Partialdruck des Fremdgases und damit auch auf die Konzentration eines beliebigen Fremdgases wie zb Stickstoff oder Lachgas geschlossen werden.

Fig. 1 zeigt die Unterschiede im Druckverlauf von $CO_2$ und dem Fremdgas Stickstoff bei der Expansion unterschiedlicher Volumina. Die spezifische Löslichkeit von N2 in Getränken wie Wasser oder Bier ist viel geringer als jene von $CO_2$. Mit einem Druck von 1 bar kann in 1 Liter Wasser 1 Liter $CO_2$ unter Normdruck gelöst werden, mit selbem Druck aber nur 0,17 Liter Stickstoff. Damit ist der größte Anteil vom Stickstoff bei der Messung mittels Expansionsmethode bei z.B. 10% Expansion bereits in der Gasphase, der Einfluss durch den Stickstoff steigt also mit geringen Expansionsvolumen. Die Stickstoff und $CO_2$-Konzentration in der Flüssigkeit sinkt. Der Partialdruck des Stickstoffes sinkt in der expandierten Kammer gegenüber dem $CO_2$-Druck rasch ab, sodass der größte Teil des Fremdgases Stickstoff sozusagen aus der Lösung diffundiert ist. Der Partialdruck ändert sich sodann nur mehr gering.

Die sich einstellende Partialdrücke durch eine Volumen-Vergrößerung v können mit einer abgewandelten Formel von Henry und Boyle wie nachfolgend dargestellt berechnet werden. Um die Formel zu vereinfachen wird mit der Löslichkeit in "Volumen/bar" statt "g/l/bar" gerechnet.

$$p'_{CO2} = p_{CO2} / (1 + v / L_{CO2})$$

$$p'_{FG} = p_{FG} / (1 + v / L_{FG})$$

$p'_x$ Partialdruck des Gases nach der Messkammer-Volumen-Vergrößerung

$p_x$ Sättigungsdruck des Gases in der Flüssigkeit

Volumenvergrößerung v = 0,1 (=10%)

$L_X$ Löslichkeit des Gases in der Probeflüssigkeit in "Volumen/bar"

Es gilt:

$$p'_{gesamt} = p_{CO2} / (1 + v / L_{CO2}) + p_{FG} / (1 + v / L_{FG})$$

$$p_{FG} = (p'_{gesamt} - p_{CO2} /(1 + v / L_{CO2})) * (1 + v / L_{FG}))$$

Für die spezifische Löslichkeit können Literaturwerte für die zu untersuchenden Fluide und Gase eingesetzt werden.

$$c_{FG} = \xi_{FG}(T)*p_{FG}$$

Im Nachfolgenden wird ein erfindungsgemäßes Verfahren anhand eines Ausführungsbeispiels von CO2 als Messgas und Stickstoff als Fremdgas exemplarisch dargestellt:

Der erste als optischer Sensor ausgebildete Sensor Carbo 520, von Anton Paar (https://www.anton-paar.com/at-de/produkte/details/carbo-520-optical/) misst die $CO_2$-Konzentration ohne Fremdgaseinfluss. Ein zweiter Sensor, der als manometrischer Sensor Carbo 510 (https://www.anton-paar.com/corp-de/produkte/details/carbo-510-smart-sensor/) ausgebildet ist und nach der Expansionsmethode arbeitet, wird zur Fremdgasbestimmung, also zur Bestimmung des Stickstoffes verwendet.

Die Volumenvergrößerung des zweiten Sensors ist mit rund 10% festgelegt, um Messfehler möglichst klein zu halten. Eine Verkleinerung hätte den Vorteil, dass ein Fehler des optischen Messwertes weniger Einfluss hat, aber dafür der Einfluss der Volumenvergrößerung bzw. Druckabhängigkeit steigt. Die Wahl des Expansionsvolumens für den manometrischen Sensor wird bei diesem Ausführungsbeispiel vorteilhaft also auf Basis der tatsächlichen spezifischen Löslichkeit des zu untersuchenden Fremdgases optimal gewählt. Das Expansionsvolumen kann alternativ für unterschiedliche Gase auch an diese angepasst und auch bei dem dargestellten Ausführungsbeispiel anders gewählt werden.

Aus der durch den ersten Sensor ermittelten $CO_2$-Konzentration ohne Fremdgaseinfluss kann der CO2 Sättigungsdruck berechnet werden:

$$p_{CO2} = c_{(CO2)OPT} / L_{CO2}$$

Der zweite Sensor liefert neben dem scheinbaren CO2-Wert auch den Gesamtdruck nach Volumen-Vergrößerung: $p'_{gesamt}$. Aus beiden Werten kann der Partialdruck des Fremdgases, hier beispielhaft Stickstoff, ermittelt werden.

$$p_{N2} = (p'_{gesamt} - p_{CO2} / (1 + v / L_{CO2})) * (1 + v / L_{N2})$$

Für die spezifische Löslichkeit können Literaturwerte für die zu untersuchenden Prozessflüssigkeiten eingesetzt werden.

$$ci = \xi i(T) * pi$$

Es gilt:
Löslichkeiten L bei einer bestimmten Messtemperatur z.B. 10°C:
$L_{CO2}$ = 1.058 bar -1, $\xi_{CO2}$ = 2.06 g/L/bar
$L_{N2}$ = 0.017 bar-1, $\xi_{N2}$ = 20 mg/L/bar
Sättigungsdrucke in der Gasphase: $p_{CO2}$ = 2. 50 bar, $p_{N2}$ = 2. 00 bar
Für Stickstoff als Fremdgas ergibt sich somit durch Umformen und Einsetzen:

$$p'_{gesamt} = p_{CO2} / (1 + v / L_{CO2}) + p_{N2} / (1 + v / L_{FG}) = 2{,}284 + 0{,}291 = 2{,}574 \text{ bar}$$

$$p_{N2} = (p'_{gesamt} - p_{CO2} / (1 + v / L_{CO2})) * (1 + v / L_{N2}) = 0{,}290 * 6{,}882 = 2 \text{ bar}$$

$$c_{N2} = \xi_{N2} * p_{N2} = 20 * 2 = 40 \text{ mg/L}$$

Eine alternative Berechnung des Gehalts des Fremdgases kann auch mithilfe eines empirisch ermittelten Polynoms aus $CO_2$, scheinbarem $CO_2$ und Temperatur erfolgen. Die durch den ersten und den zweiten Sensor ermittelten $CO_2$ Gehalte bzw die daraus berechneten Partialdrücke werden hierfür herangezogen. Die Differenz der gemessenen Druckwerte wird durch Messung bekannter Proben mit bekannten Gehalten an $CO_2$ und einem bestimmten Fremdgas untersucht. Die unterschiedlichen Messwerte werden mit den in Standardmessgeräten, z.B. dem CarboQC der Anmelderin oder anderen hochgenauen Laborgeräten, vermessen.

Die Differenz der so ermittelten CO2-Konzentrationen wird ermittelt, aus der Differenz der $c_{DEV} = c_{(CO2)Man} - c_{(CO2)OPT}$

Aus dieser Differenz wird dann der Fremdgasgehalt zB Stickstoffgehalt oder Lachgas ermittelt und eine Tabelle mit Fremdgaswerten zur jeweiligen $c_{DEV}$ bei verschiedenen Temperaturen in der Auswerteeinheit hinterlegt. In der Prozesslinie werden dann die gemessenen Differenzen aus einer entsprechenden Look - up Tabelle ausgelesen.

Die Messwerte können optional auch mit einem Polynom höherer Ordnung gefittet werden und als Berechnungskurven in der Auswerteeinheit hinterlegt werden. Dabei werden die entsprechenden Löslichkeiten Temperaturen und Druckdifferenzen ausgewertet und berücksichtigt

$C_{N2} = f(c_{DEV}, c_{(CO2)OPT}, t)$

So kann alternativ der Zusammenhang zwischen Messgaskonzentration ohne Fremdgaseinfluss und Messgaskonzentration mit Fremdgaseinfluss vorab werkseitig anhand von Messungen an bekannten Proben bestimmt wird. Die Werte für die Fremdgaskonzentration werden dann als, insbesondere temperaturabhängige, Konzentrationsdifferenz des Messgases als Berechnungskurven und/oder Tabellenwerte in der Auswerteeinheit der Messgeräte zur Verfügung gestellt bzw in dieser hinterlegt. Bei der Messung der Konzentrationen bzw des Drucks und der Temperatur wird dann die Fremdgaskonzentration anhand dieser Werte bei der Messung ermittelt.

Die Anwendbare Formel ist dann nicht universal sondern jeweils nur für eine Prozessflüssigkeit, z.B. Bier, gültig und wird je nach zu messender Prozessflüssigkeit für z.B. Soft Drinks angepasst und eine entsprechende Formel entwickelt bzw. in der Auswerteeinheit hinterlegt. Der Benutzer kann also in der Auswerteeinheit dir untersuchte Prozessflüssigkeit zb. Bier, Cola, Diätcola,..., sowie das Fremdgas auswählen und damit die richtige Auswertekurve heranziehen.

Die Auswerteeinheit kann dabei jeweils in jedem Sensor selbst vorliegen, also die Messwerte in einer dem Sensor zugeordneten bzw. in diesem angeordneten Auswerteeinheit direkt verarbeitet werden oder einer zentralen Auswerteeinheit zugeführt werden in der die Messwerte der Sensoren dann verarbeitet werden und die Bestimmung der Konzentration des Fremdgases erfolgt.

Optional kann die Genauigkeit auch bei geringem $CO_2$-Gehalt in der Flüssigkeit verbessert werden. Wird die Prozessflüssigkeit bei geringen $CO_2$-Konzentrationen in der Prozessflüssigkeit expandiert, so ergibt sich der einstellende Druck besonders bei großer Expansion nicht nur aus dem $CO_2$-Gas aus der Prozessflüssigkeit, sondern auch aus dem Wasserdampf der wässrigen Lösung. Um die Genauigkeit dann zu erhöhen, wird auch der Dampfdruck des Wassers bei der Bestimmung der Konzentration des Fremdgases berücksichtigt, der bei der Messung des Prozessgases beispielsweise der $CO_2$-Messung ermittelte Druckwert wird deshalb korrigiert:

$$p_{kor} = p_{Gas} - p_{Dampf}$$

Dies passiert mit einer Formel, die Magnus Formel, die der Literatur entnommen werden kann und die Abhängigkeit des Dampfdrucks von der Messtemperatur berücksichtigt. (Magnusformel für den Sättigungsdampfdruck über ebenen Wasseroberflächen:)

$$p_{Dampf} = 0.006112 * Exp(17.62 * t / (t + 243.12) \text{ (für } -45°C < T < 60°C)$$

Weiters kann die Genauigkeit des erfindungsgemäßen Verfahrens erhöht werden in dem der Realgasfaktor oder der Kompressibilitätsfaktor herangezogen werden. Der Realgasfaktor oder Kompressibilitätsfaktor beschreibt die Abweichung eines realen Gases vom idealen Verhalten. Die Gase weichen bei endlichem Volumen und höheren Drücken zum Teil erheblich vom idealen Verhalten ab.

$m_G = p*V_H*M/(R*T*Z)$ Masse des Gases im Kopfraum

p ... $CO_2$ Partialdruck

$m_G$ ... Masse $CO_2$

$V_H$ ... Volumen Kopfraum

M ... Molmasse

R ... Gaskonstante

T ... Temperatur in K

Z ... Kompressibilität

$$Z = 1.0005 + p_{kor} * (-0.007 + T * 0.0000674)$$

Z ist der Kompressibilitätsfaktor des $CO_2$, dieser ist Temperatur- und Druckabhängig und kann prinzipiell der Literatur entnommen werden. Für den Anwendungsfall liegt der Faktor in der Größenordnung von 0,98 und spielt umso mehr eine Rolle je größer das Volumen der Expansion ist.

Fig, 2 zeigt die Ergebnisse eines Beispiels der Stickstoffmessung mit der Kombination aus einem ersten optischen Sensor Carbo520 und einem zweiten manometrischen Sensor Carbo510.

Das System wurde mit Luftgesättigtem Wasser gestartet, das über einen Wärmetauscher mit Thermostat auf 20° C gekühlt wird.

Zu zwei Zeitpunkten t1 13:25 (1:25 PM) und t2 14:25 (2:25 PM) wurde jeweils Stickstoff in das untersuchte Fluid injiziert: Die bestimmte Stickstoffkonzentration korreliert mit dem Sättigungsdruck.

Fig, 3 zeigt die Ergebnisse eines Beispiels der Distickstoffmonoxid-Messung (Lachgas) mit der Kombination aus einem ersten optischen Sensor Carbo520 und einem zweiten manometrischen Sensor Carbo510.

Das System wurde mit Wasser als Prozessflüssigkeit gestartet, das über einen Wärmetauscher mit Thermostat auf 20° C gekühlt wird.

Zu zwei Zeitpunkten t1 14:38 (2:38 PM) und t2 15:05 (3:05 PM) wurde jeweils Distickstoffmonoxid in die untersuchte Prozessflüssigkeit injiziert. Die bestimmte Distickstoffmonoxid -Konzentration korreliert mit dem Sättigungsdruck.

Alternativ zum ersten Ausführungsbeispiel kann beispielsweise auch der erste und/oder der zweite Sensor innerhalb eines Behälters angeordnet sein, in dem die Prozessflüssigkeit gesammelt wird. In derartigen Behältern beispielsweise Getränketanks wird die Prozessflüssigkeit beispielsweise Bier gesammelt und es bildet sich eine Gasphase, der sogenannte Kopfraum, über der Prozessflüssigkeit aus, in dem das Messgas im ungelösten Anteil vorliegt. So liegen die Prozessflüssigkeit, das Messgas und das Fremdgas in dem Behälter im zweiphasigen Zustand vor, wobei die Prozessflüssigkeit mit gelösten Anteilen des Messgases und des Fremdgases die erste flüssige Phase bilden und über diesen im Kopfraum ein ungelöster Anteil des Messgases und des Fremdgases in einem Gasgemisch die zweite Phase ausbilden. Hierbei können erfindungsgemäß der erste Sensor und der zweite im Bereich des Behälters angeordnet sein indem das Messgas und das Fremdgas in der Prozessflüssigkeit gelöst vorliegen.

Das erfindungsgemäße Verfahren kann dann, wie zum ersten Ausführungsbeispiel dargelegt, erfindungsgemäße durchgeführt werden.

Alternativ kann ebenfalls vorgesehen sein, dass das Fremdgas erst im Laufe der Produktion der Prozessflüssigkeit, beispielsweise des Bieres, hinzugefügt wird. So kann alternativ zuerst der Gehalt des Messgases beispielsweise Kohlendioxid mittels des ersten $CO_2$-Sensors beispielsweise über die ATR-Methode bzw. einen ATR-Sensor ermittelt werden, um so den exakten $CO_2$-Gehalt innerhalb der Prozessflüssigkeit zu bestimmen. In einem weiteren Schritt wird dann das Fremdgas zum Beispiel Stickstoff hinzugefügt und anschließend die Messung mit dem zweiten Sensor beispielsweise einem manometrischen Sensor durchgeführt und dann über das erfindungsgemäße Verfahren über die gemessenen Drücke bzw. bestimmten Partialdrücke des Messgases und des Fremdgases ermittelt.

Eine weitere alternative Ausführungsform der erfindungsgemäßen Vorrichtung bzw. des erfindungsgemäßen Verfahrens sieht vor, dass nur ein Sensor innerhalb der Prozesslinie bzw. dem Behälter vorgesehen ist und zuerst das Messgas vor Zugabe eines Fremdgases bestimmt wird und anschließend die mit dem Fremdgas versetzte Prozessflüssigkeit wieder an dem Sensor vorbeigeführt wird bzw. mit diesen Sensor eine zweite Messung durchgeführt wird und derart auf Basis der unterschiedlichen Messwerte der hinzugefügte Anteil des Fremdgases bestimmt wird.

Besonders bevorzugt ist bei der Ausbildung der Vorrichtung mit zwei Sensoren bzw. der Durchführung des erfindungsgemäßen Verfahrens mit zwei Sensoren, dass diese beiden Sensoren bzw. die Messpunkte der exakten $CO_2$-Bestimmung und der Bestimmung des Gesamtdruckes bzw. Temperatur in geringem Abstand zueinander erfolgen bzw. die beiden Sensoren im Leitungssystem einander gegenüber angeordnet sind.

## Patentansprüche

1. Verfahren zur Bestimmung des Gehalts eines Fremdgases in einer Prozessflüssigkeit, in dem ein Messgas, insbe-sondere $CO_2$, gelöst ist,
   wobei die Konzentration des Messgases ermittelt wird,

   - wobei die Konzentration des durch das Messgas und das Fremdgas gebildeten Gasgemischs, insbesondere über eine manometrische Messmethode, ermittelt wird,
   - wobei die Messwerte einer Auswerteeinheit zugeführt werden, - wobei anhand der ermittelten Konzentration des Messgases und der ermittelten Konzentration des Gasgemisches die Konzentration des Fremdgases bestimmt wird.

2. Verfahren nach Anspruch 1, **dadurch gekennzeichnet, dass** der Gesamtdruck und die Temperatur des durch das Messgas und das Fremdgas gebildeten Gasgemischs, insbesondere über eine manometrische Messmethode, ermittelt wird,

   - wobei anhand der ermittelten Konzentration des Messgases und des ermittelten Gesamtdrucks und der ermittelten Temperatur der Partialdruck des Messgases bestimmt wird,
   - wobei der Partialdruck des Fremdgases anhand des ermittelten Partialdrucks des Messgases und anhand des gemessenen Gesamtdrucks und der gemessenen Temperatur ermittelt wird, und
   - wobei über den Partialdruck des Fremdgases die Konzentration und/oder der Gehalt des Fremdgases bestimmt wird.

3. Verfahren nach einem der vorangehenden Ansprüche, **dadurch gekennzeichnet, dass** nach der Ermittlung der Konzentration des Messgases der Prozessflüssigkeit Fremdgas hinzugefügt wird.

4. Verfahren nach Anspruch 2, **dadurch gekennzeichnet, dass** die Konzentration des Messgases mittels eines manometrischen Sensors über eine manometrische Messmethode, insbesondere über eine Expansionsmethode, bevorzugt mit einer Volumsvergrößerung von 1% bis 20%, besonders bevorzugt mit einer Volumsvergrößerung von 3%- 10%, ermittelt wird.

5. Verfahren nach Anspruch einem der vorangehenden Ansprüche, **dadurch gekennzeichnet, dass** die Konzerntration des Messgases mittels eines ersten Sensors ermittelt wird, der als optischen Sensor, insbesondere als ATR-Sensor, ausgebildet ist, wobei mittels des ersten Sensors insbesondere eine optische Absorptionsmessung des Messgases durchgeführt wird und/oder
dass der Gesamtdruck und die Temperatur des durch das Messgas und das Fremdgases gebildete Gasgemischs mittels eines zweiten Sensors ermittelt wird, der als manometrischer Sensor ausgebildet ist und der Gesamtdruck über eine manometrische Messmethode, insbesondere über eine Messung nach der Druck-Temperatur-Methode oder Volumenexpansionsmethode, ermittelt wird.

6. Verfahren nach Anspruch 5, **dadurch gekennzeichnet, dass** der Partialdruck des Fremdgases ($P_F$) mithilfe der Gleichung $P_F = (p'_{gesamt} - p_{MG} / (1 + v / L_{MG})) * (1 + v / L_F)$ ermittelt wird.

7. Verfahren nach einem der vorangehenden Ansprüche, **dadurch gekennzeichnet, dass** das Messgas Kohlendioxid und das Fremdgas insbesondere Stickstoff oder Distickstoffmonoxid ist und/oder
dass bei der Bestimmung der Konzentration des Messgases und/oder des Fremdgases die Löslichkeit und/oder die Kompressibilität des Messgases und/oder des Fremdgases in der Prozessflüssigkeit berücksichtigt wird.

8. Verfahren nach einem der vorgehenden Ansprüche, **dadurch gekennzeichnet dass** die Differenz der Messwerte der beiden Sensoren mit Proben bekannter Konzentration von Messgas und Fremdgas kalibriert werden und diese Kalibrationskurven oder Tabellen in der Auswerteeinheit hinterlegt werden.

9. Verfahren nach einem der vorangehenden Ansprüche, **dadurch gekennzeichnet,**

   - **dass** der Zusammenhang zwischen der Konzentration des Messgases ohne Fremdgaseinfluss und der Konzentration des Gasgemisches bestehend aus Messgas und Fremdgas vor Beginn der Messung anhand von Messungen an bekannten Proben bestimmt wird,
   - **dass** die Werte für die Fremdgaskonzentration als, insbesondere temperaturabhängige, Konzentrationsdifferenz des Messgases als Berechnungskurven und/oder Tabellenwerte in der Auswerteeinheit der Messgeräte zur Verfügung gestellt werden, und
   - **dass** die Fremdgaskonzentration anhand dieser Werte bei der Messung ermittelt wird.

10. Verfahren nach einem der vorangehenden Ansprüche, **dadurch gekennzeichnet, dass** die Bestimmung der Konzentration des Messgases und die Messung des Gesamtdruckes und Temperatur des Gasgemisches in einem Leitungssystem der Prozessflüssigkeit oder einem Behälter, insbesondere in geringem Abstand zu einander, bevorzugt einander gegenüberliegend, erfolgt.

11. Verfahren nach einem der vorangehenden Ansprüche, **dadurch gekennzeichnet, dass** die Prozessflüssigkeit ein Getränk mit gelösten Gasen, insbesondere Bier, ist, und/oder
dass bei der Bestimmung des Gehalts des Messgases der Gehalt von in der Prozessflüssigkeit enthaltener Inhaltsstoffe wie Extrakte und Alkohol berücksichtigt wird und/oder
dass bei der Bestimmung der Konzentration des Messgases und des Fremdgases der Dampfdruck der Prozessflüssigkeit berücksichtig wird.

12. Verfahren nach einem der vorangehenden Ansprüche, **dadurch gekennzeichnet, dass** die Prozessflüssigkeit, das Messgas und das Fremdgas in einem Behälter in zweiphasigem Zustand vorliegen, wobei die Prozessflüssigkeit mit gelösten Anteilen des Messgases und des Fremdgases die erste Phase bilden und ein ungelöster Anteil des Messgase und des Fremdgases als Gasgemisch die zweite Phase ausbilden, wobei der erste Sensor und der zweite im Bereich des Behälters angeordnet sind indem das Messgas und das Fremdgas in der Prozessflüssigkeit gelöst vorliegen.

13. Vorrichtung zur Bestimmung des Gehalts eines Fremdgases in einer Prozessflüssigkeit in der ein Messgas gelöst ist, wobei die Vorrichtung ein Leitungssystem aufweist, in dem die Prozessflüssigkeit fließen kann und/oder die Vorrichtung einen Behälter aufweist in dem die Prozessflüssigkeit sammelbar ist, **dadurch gekennzeichnet,**

- **dass** die Vorrichtung einen ersten Sensor aufweist, mit dem der Gehalt des Messgases ermittelbar ist, und
- **dass** die Vorrichtung einen, insbesondere zweiten, Sensor aufweist, mit dem der Gesamtdruck und die Temperatur des Messgases und des Fremdgases in der Prozessflüssigkeit, insbesondere über eine manometrische Messmethode, ermittelbar ist, und
- **dass** die Vorrichtung eine Auswerteeinheit aufweist, die zur Durchführung des Verfahrens nach einem der Ansprüche 1 bis 12 ausgebildet ist.

14. Vorrichtung nach Anspruch 13, **dadurch gekennzeichnet, dass** der Sensor als optischer Sensor, insbesondere als ATR-Sensor, ausgebildet ist, und/oder dass der Sensor, insbesondere der zweite Sensor, als manometrischer Sensor ausgebildet ist, und insbesondere derart ausgebildet ist, dass die Messung nach der Druck-Temperatur-Methode oder Volumenexpansionsmethode mittels des Sensors durchführbar sind.

15. Vorrichtung nach einem der Ansprüche 13 bis 14, **dadurch gekennzeichnet, dass** der erste Sensor und der zweite Sensor in dem Leitungssystem in geringem Abstand zueinander, insbesondere gegenüber voneinander, angeordnet sind, und insbesondere über Flansche in dem Leitungssystem anbringbar sind, und/oder dass der erste Sensor und der zweite Sensor im Behälter gegenüber einander angeordnet sind.

Fig. 1

Fig. 2

Fig. 3

Europäisches Patentamt
European Patent Office
Office européen des brevets

# EUROPÄISCHER RECHERCHENBERICHT

Nummer der Anmeldung

EP 21 17 1763

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 762 837 A2 (HAFFMANS BV [NL]) 14. März 2007 (2007-03-14) | 1,2,5,6, 8,9, 11-14 | INV. G01N7/14 G01N33/14 |
| Y | * Abbildung 2 * * Absätze [0003], [0017] - [0018], [0025], [0030] - [0036] * | 3 | ADD. G01N21/552 |
| X | US 2003/029228 A1 (BLODER JOSEF [AT] ET AL) 13. Februar 2003 (2003-02-13) | 1,2,4, 7-13,15 | |
| Y | * Abbildung 1 * * Absätze [0025] - [0038], [0108] * | 3 | |
| Y | US 2013/340497 A1 (TATA MURTHY [US]) 26. Dezember 2013 (2013-12-26) * Anspruch 1 * * Absätze [0008] - [0009], [0013], [0055] * | 3 | |
| A | EP 1 630 543 A1 (METTLER TOLEDO GMBH [CH]) 1. März 2006 (2006-03-01) * Absätze [0004], [0009], [0015] * | 5,14 | |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| Den Haag | 7. September 2021 | Bockstahl, Frédéric |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 17 1763

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-09-2021

| Im Recherchenbericht angeführtes Patentdokument | Datum der Veröffentlichung | Mitglied(er) der Patentfamilie | Datum der Veröffentlichung |
|---|---|---|---|
| EP 1762837 A2 | 14-03-2007 | EP 1762837 A2<br>NL 1029934 C2 | 14-03-2007<br>16-03-2007 |
| US 2003029228 A1 | 13-02-2003 | AT 409673 B<br>CH 696086 A5<br>DE 10213076 A1<br>GB 2373584 A<br>US 2003029228 A1 | 25-10-2002<br>15-12-2006<br>26-09-2002<br>25-09-2002<br>13-02-2003 |
| US 2013340497 A1 | 26-12-2013 | US 2013340497 A1<br>US 2016054278 A1 | 26-12-2013<br>25-02-2016 |
| EP 1630543 A1 | 01-03-2006 | KEINE | |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- AT 409673 B

- AT 512291 B1

**In der Beschreibung aufgeführte Nicht-Patentliteratur**

- Die manometrische Bestimmung des CO2-Gehalts in Getränken. *Brauwelt,* 1991, vol. 50, 2402